# EUROPEAN PATENT APPLICATION

(11) **EP 3 878 385 A1**
(43) Date of publication of application: **15.09.2021**
(21) Application number: 19882034.2
(22) Date of filing: 07.11.2019
(51) Int. Cl.: A61B 17/42, A61M 25/10

(54) **INTRAUTERINE EXPANDER DEVICE**

(30) Priority: 08.11.2018 ES 201831078
(71) Applicant: Alonso Pacheco, Luis, 29630 Benalmadena (ES); Haimovich, Yaffa, 08197 Sant Cugat del Valles (ES); Sabaris Vilas, Joaquin, 08213 Polinya del Valles (ES)
(72) Inventor: SABARÍS VILAS, Joaquín, 08213 Polinya Del Valles (ES)
(74) Representative: Toro Gordillo, Ignacio Maria
(86) International application number: PCT/ES2019/070759
(87) International publication number: WO 2020/094903

(57) **Abstract**

The invention consists of an expansion catheter for intrauterine placement, featuring anatomical characteristics adaptable to the different morphologies of the uterine organ in its internal cavities, and which efficiently enables the dilatation and separation of the internal walls of the cavity subsequent to their reduction in the surgical process of endometrial scraping in the different surgical techniques of the procedure, in order to serve as a separator and isolator in the prevention of intrauterine adhesions, facilitating drainage both internally through the device and perimetrally. The device, containing an expansion device in a collapsed state, is placed in the uterus by means of an insertion device with the aid of a pusher, which is discarded together with the insertion device once the unit is in place.

## Description

### OBJECT OF THE INVENTION

The present invention relates to an expander catheter for intrauterine placement with anatomical features adaptable to the different morphologies presented by the uterine organ in its inner cavities and which efficiently allows for expansion and separation of the inner walls of the uterine cavity in order to fulfil a dual purpose: on one hand to separate the uterine walls in order to prevent intrauterine adhesions, i.e. avoiding the coaptation thereof, and on the other hand to allow compression of the uterine walls, acting as a hemostatic factor.

### BACKGROUND OF THE INVENTION

The uterine cavity is what is defined in medicine as a virtual cavity. This means that the uterine walls, in the absence of artificial separation between them, are in intimate contact. This coaptation of the uterine wall may lead to adhesion formation or scarring that occurs between the opposite faces in cases where an alteration or internal injury occurs, such as in the case of intrauterine surgery.

There are various methods that have been used over the years aimed at reducing the rate of formation of these adhesions. Among the different methods, physical or barrier methods are the most used and effective, as evidenced by the comparative studies of *Lin (*Lin, X., Wei, M., Li, T. C., Huang, Q., Huang, D., Zhou, F., & Zhang, S. (2013). A comparison of intrauterine balloon, intrauterine contraceptive device and hyaluronic acid gel in the prevention of adhesion reformation following hysteroscopic surgery for Asherman syndrome: a cohort study. European Journal of Obstetrics & Gynecology and Reproductive Biology, 170 (2), 512-516. doi: 10.1016 / j.ejogrb.2013.07.018*),* which observed that the rate of adhesion when an intrauterine catheter was used was lower than when an IUD or antiadherential gel was used.

These types of catheters are embodied in a kind of inflatable body that involves a series of extracorporeal outer tubes through which, once implanted, the device itself can inflate, defining a single central drainage channel.

The problem with this type of device is its design (badly adapted to the uterine anatomy), the difficulty of placement and the poor drainage of accumulated bleeding inside the cavity.

The invention also relates to the method of Patent WO2016074647, wherein an inflatable balloon species is provided for separating the uterine walls, and balloons for fixing to the cervix, with two push valves, also having the same problems of the difficulty in the placement and the poor drain of the bleeding accumulated inside the cavity, by providing only an internal drain.

In parallel, in US pat. No. 4552557A, there is also provided an inflatable balloon species, also with an inner drain, in which a single internal inflation chamber is involved in the form of a triangle, with the same limitations from the point of view of problems relating to the difficulty in placement and the poor drain of the bleeding accumulated inside the cavity, by providing only an internal drain.

The invention also relates to these problems in addition to the fact that these devices do not provide A guidance system for the introduction of the catheter with fixation to the uterine and posterior base with lateral drainage to the cervix.

### DESCRIPTION OF THE INVENTION

The intrauterine expander device that is proposed solves the problems of transvaginal placement and internal positioning, and also avoids the patient's discomfort due to over inflation of the walls, presenting substantial improvements in its many drainage channels for removal of additional pressure caused by liquid accumulation and the obstruction of the main drainage channel, in addition to not having extracorporeal outer tubes.

To that end and more specifically, based on the conceptual structure of the type of devices described above, the device of the invention presents the particularity that it is embodied from an inflatable main body, which has a configuration in the form of an inverted spear tip with a blunt tip in its central area, coinciding with a central drainage channel with a drain through a central tube, with the particularity that said body is formed from several superimposed individual inflation chambers, which determine two flexible lateral flaps with rounded edges which are initially folded occupying a minimum volume within an insertion device, of tubular and open configuration, like a sheath. Thus, it substantially facilitates implanting the device and its passage through the cervix, securing the depth of the end sites in the introduction of the insertion device, and which can be easily removed coaxially from the device once arranged in the work area by means of an internal pusher which is also discarded together with the insertion device which can be easily deployed by selective inflation of its chambers, for which the same are associated to small inflation tubes passing internally to the main drainage tube, having a length greater than the latter, so that at their lower extremity they adopt a spiral configuration allowing their retraction once the device is adapted to the physiognomy of the patient, and which are intracavitary, not external, to improve the discomfort of wearing them hanging on the outside, thus avoiding possible sources of infection and limitation of the patient's activity, said tubes concluding in the corresponding non-return valves.

Thus, the main inflatable body presents an ergonomic configuration with variables adapting to the different morphologies of the organ, defining, when inflated, a contour section curved both in its two major faces and its two minor faces, which determine in its support on the uterine wall a plurality of additional drainage conduits to the central drainage channel.

More specifically, in the main body a double centre and lateral inflation quadruple chamber to create more drainage channels is involved; apart from the unified central channel, thus covering all angles of liquid removal by intra-mural secretion and thus avoiding over pressures and epithelial tissue adhesions.

In turn, said main body ends lower down in a triple balloon for attaching to the cervix, with discontinuous lateral attachment rings and drainage canalisation to prevent its dislodgement and possible withdrawals during the phases of muscle contraction.

Finally, after the triple balloon emerges the aforementioned central drainage tube, which extends to the upper edge as a complement to the different inflation channels, ie, in addition to the lateral drainage channels which are formed upon inflation of the chambers, a central conduit is added for the evacuation of internal liquids, thus defining seven drain channels, one central, two upper, two lower and two lateral channels.

The special configuration of the device causes it to have a smaller size and weight, without external tip shaped plastic forms.

Similarly, having different inflation chambers allows partial and irregular inflation for greater adaptation to intracavitary dysmorphies.

As for the main body folding mode within the sheath, it will be folded as a pivoting shaft for more efficient deployment in the catheter adaptability to the lateral walls of the uterine cavity.

The morphology of the device deployed in the form of a lateral float with internal canalisation paths and the frontal inflation limiter allow greater adaptability to the intrauterine cavities, as well as a greater volume of separation, without any inherent discomfort in placement, to avoid possible total or partial adhesions that involve a subsequent re-intervention by the gynaecologist; discomfort added to the phases of muscle contraction of the uterus in the recovery stage.

### DESCRIPTION OF THE DRAWINGS

To complete the description that is going to be made and to assist a better understanding of the invention's characteristics, according to a preferred practical embodiment thereof, accompanying as an integral part of said description, is a set of drawings, where in an illustrative and non limiting way, the following is represented:

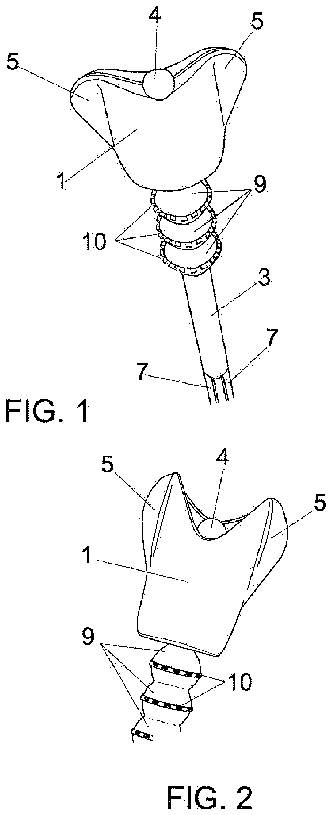

Figure 1 shows a top side perspective view of an intrauterine expander device made in accordance with the object of the present invention, deployed but not fully inflated.
Figure 2 shows a detail in a different perspective from the whole of the previous figure.

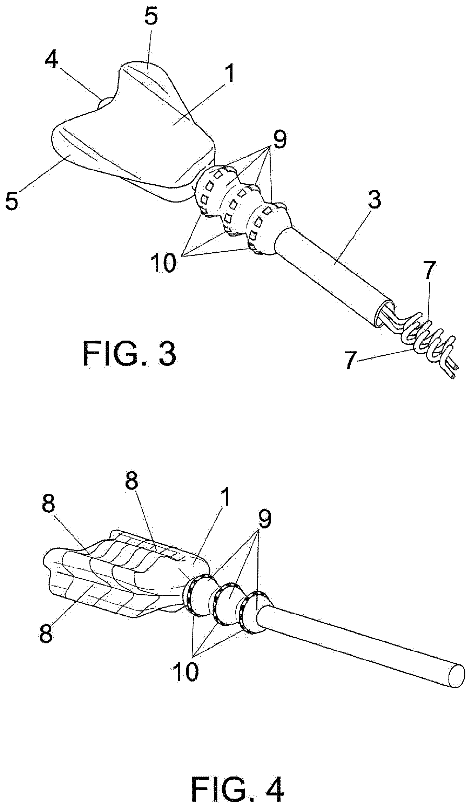

Figure 3 shows the device of Figure 1 according to another perspective.
Figure 4 shows a perspective view of the device of the preceding figures, duly inflated.
Figure 5 finally shows a sectional view of the device, introduced into an applicator sheath for the introduction of the assembly into the uterus.

### PREFERRED MODE FOR CARRYING OUT THE INVENTION

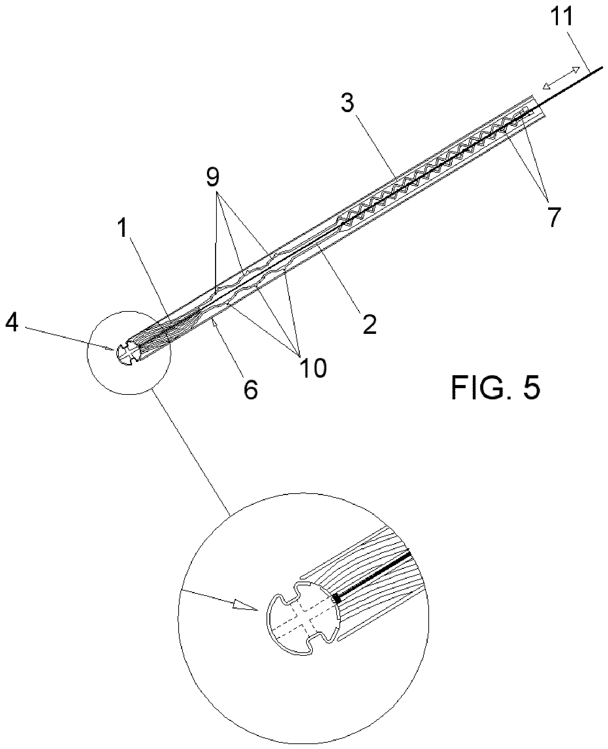

In view of the aforementioned figures, it can be seen that the proposed intrauterine expander device is constituted from an inflatable main body (1), which has a configuration in the form of an inverted spear tip, with rounded edges, in which a central drainage channel (2) towards a central tube (3) is defined above, which central channel (2) has a blunt tip (4), such that said inflatable main body (1) is constituted from several individual inflation chambers, which determine two flexible lateral flaps (5) with rounded edges that initially fold occupying a minimum volume within an inserter device (6), the one shown in Figure 5, of tubular configuration with rounded and open tip (11), like a sheath, which significantly facilitates the implantation of the device and its passage through the cervix.

This inserter device (6) can be easily coaxially removed from the main device by pulling it, once arranged in the work area, next to the internal pusher element (11).

In this way, the device can be easily deployed by inflating its chambers, for which they are associated with small inflation tubes (7) that pass internally to the central drainage tube (3), presenting a greater length than the latter, so that in their lower extremity, not shown in the figures, they adopt a spiral configuration, which allows their retraction once the device is adapted to the physiognomy of the patient, ending at these extremities in the corresponding non-return valves, not represented in the figures.

The main body (1) presents an inflatable and ergonomic configuration adaptable to the different morphologies of the organ, with the particularity that, when inflate, it defines a contour section curved both in its two major faces and its two minor faces, which determine, in its support on the uterine wall, a plurality of drainage conduits or channels (8) additional to the central drainage channel.

In this sense, and as noted above, a double centre and lateral inflation quadruple chamber is involved in the main body to create a greater number of drainage channels (8).

Lower down the main body (1) a triple balloon for attaching to the cervix (9), with discontinuous lateral attachment rings and drainage canalisation to prevent its dislodgement and possible withdrawals during the phases of muscle contraction, is arranged.

From this structuring, the following structural advantages are derived:
- Device with its own inserter, which significantly facilitates its placement and reduces the discomfort of said operation.
- The multi-chamber configuration allows for greater expansion along the lateral edges.
- Anatomical shape that adapts to the uterine cavity.
- Wide distension capacity, which increases the separation of the walls exerting hemostatic action by compression.
- Increased perimeter drainage capacity of accumulated secretions.

## Claims

1. Intrauterine expander device, **characterised in that** it is constituted from an inflatable main body (1), which has a configuration in the form of an inverted spear tip, with rounded edges, in which a central drainage channel (2) is defined above towards a central tube (3), which central channel (2) has a blunt tip (4), such that said inflatable main body (1) is constituted from several individual inflation chambers, which determine two flexible lateral flaps (5) with rounded edges, said body having on its inflation a contour section curved both on its major faces and on its minor faces, which determine in its support on the walls of the uterus, a plurality of drainage conduits or channels (8) additional to the central drainage channel determined by the central tube (3), it having been provided that the chambers of the inflatable main body (1) are assisted by small inflation tubes (7) that pass internally to the central drainage tube (3), with the particularity that below the main body (1) a series of balloons, preferably three, attached to the cervix (9) are arranged with discontinuous rings (10) to facilitate perimeter drainage.

2. Intrauterine expander device, according to claim 1, **characterised in that** it includes an inserter device (6) in which the main body (1), of tubular and open configuration, like a sheath, is initially inserted and folded, as an inserting element of the device through the cervix, axially removable with respect to the device once inserted with the help of an internal pushing element (11).

3. Intrauterine expander device, according to claim 1, **characterised in that** the inflation tubes (7) adopt a spiral configuration in correspondence with their lower extremity, giving them a retractable character.

4. Intrauterine expander device, according to claim 1, **characterised in that** a double centre and lateral inflation quadruple chamber is involved in the main body.
